# EUROPEAN PATENT APPLICATION

(11) **EP 2 330 099 A1**
(43) Date of publication of application: **08.06.2011**
(21) Application number: 09813105.5
(22) Date of filing: 10.09.2009
(51) Int. Cl.: C07D 265/30

(54) **NOVEL SALT OF MORPHOLINE DERIVATIVE**

(30) Priority: 11.09.2008 JP 2008234066
(71) Applicant: Astellas Pharma Inc., Tokyo 103-8411 (JP)
(72) Inventor: KIKUCHI, Kazumi, Tokyo 103-8411 (JP); KURODA, Akio, Tokyo 103-8411 (JP); MIZOGUCHI, Ryo, Tokyo 103-8411 (JP); SAKAMOTO, Kenichirou, Tokyo 103-8411 (JP); TAKEGUCHI, Kazuhiro, Tokyo 103-8411 (JP)
(74) Representative: Albrecht, Thomas
(86) International application number: PCT/JP2009/065792
(87) International publication number: WO 2010/029959

(57) **Abstract**

[Problem]

Provided are an acid addition salt of (+)-2-[(inden-7-yloxy)methyl]morpholine (compound A) and a novel crystal thereof, which are for use as a medicament or a drug substance, and which has high storage stability, particularly high stability against humidity and light.

[Means for Solution]

An acid addition salt of the compound A with an acid selected from benzenesulfonic acid and hydrobromic acid is a compound which is isolated in the form of a crystal, is a solid at room temperature, exhibits no hygroscopicity at such a level that causes a problem for use as a medicament or a drug substance, has stability against humidity and light, and is therefore extremely useful as a medicament or a drug substance.

## Description

### Technical Field

The present invention relates to a novel acid addition salt of (+)-2-[(inden-7-yloxy)methyl]morpholine (which is hereinafter referred to as a compound A), which is useful as a pharmaceutical, particularly as a serotonin and norepinephrine reuptake inhibitor (which is hereinafter referred to as an SNRI).

### Background Art

It is known that indeloxazine hydrochloride has a chemical structure below, and has a serotonin and norepinephrine reuptake inhibitory action and an anti-depression action. Indeloxazine hydrochloride has been used for treatment of psychiatric symptoms associated with cerebrovascular disorders such as post-stroke depression, emotional disturbance, reduced motivation, and the like in Japan and South Korea (Patent Citation 1, and Non-Patent Citations 1 and 2).

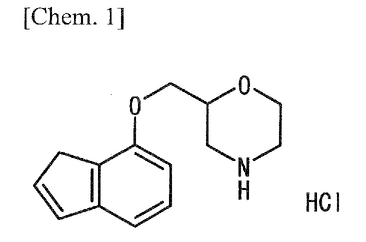

The compound A which is an optically active compound of indeloxazine has a serotonin and norepinephrine reuptake inhibitory action (Non-Patent Citation 1), and is useful against neuropathic pain and diabetic neuropathy according to international publications after the priority date of the present application (Patent Citations 2 and 3).

As an addition salt of the compound A with a pharmaceutically acceptable acid, only a hydrochloride of the compound A has been reported (Non-Patent Citation 1, and Patent Citations 2 and 3), and with respect to other addition salts with pharmaceutically acceptable acids, there is no specifically known acid addition salt.

### Prior Art

### Patent Citation

[Patent Citation 1] Specification of U.S. Patent No. 4109088
[Patent Citation 2] Pamphlet of International Patent Application Publication No. WO2008/111668
[Patent Citation 3] Pamphlet of International Patent Application Publication No. WO2008/111669

### Non-Patent Citation

[Non-Patent Citation 1] "Chemical and Pharmaceutical Bulletin", 1985, Vol. 33, No. 9, p. 3766-3774
[Non-Patent Citation 2] "Neuropharmacology" 1998, Vol. 37, p. 1169-1176

### Disclosure of Invention

### Problem to Be Solved by the Invention

It was found that a hydrochloride of the compound A has an analgesic action for restoring the reduction in pain threshold caused by neuropathy in an L5/L6 spinal nerve ligation model which is a disease model for neuropathic pain to a normal level. Indeloxazine hydrochloride is a compound which is observed to exhibit substantially no weight increase by hygroscopicity, even when stored at a relative humidity of 93% for 14 days at around room temperature, and is stable under normal conditions (room temperature, tightly closed, light shielding, 36 months). Although a hydrochloride of the compound A that is an optically active product can be obtained as an anhydride (Chemical and Pharmaceutical Bulletin 1985, Vol. 33, No. 9, p. 3766-3774), was found that it is an acid addition salt which is hygroscopic, and thus deliquescent at a relative humidity of approximately 75% (room temperature) which means a general environment, and has properties of low stability against light.
Therefore, in order to supply a more stable medicament or drug substance, it is desirable to find an acid addition salt of the compound A with a pharmaceutically acceptable acid, other than a hydrochloride, which has lower hygroscopicity, excellent stability under increased humidity conditions, and thus higher stability against light.

### Means for Solving the Problem

The present inventors have extensively studied various acid addition salts of the compound A, but there were few acid addition salts obtained, which is a salt of a pharmaceutically acceptable acid, and in the form of a solid at room temperature. Among these, it has been found that a specific acid addition salt of the compound A has excellent hygroscopicity and stability under increased humidity conditions, and excellent stability against light, as compared to the hydrochloride which is a known compound, thus completing the present invention.

Thus, an object of the present invention is to provide a salt of (+)-2-[(inden-7-yloxy)methyl]morpholine with a pharmaceutically acceptable acid selected from a group consisting of benzenesulfonic acid and hydrobromic acid.

It is another object of the present invention to provide a pharmaceutical composition comprising an effective amount of a salt of (+)-2-[(inden-7-yloxy)methyl]morpholine with a pharmaceutically acceptable acid selected from a group consisting of benzenesulfonic acid and hydrobromic acid, and further a pharmaceutically acceptable carrier.

### Effects of the Invention

The specific acid addition salt of the compound A of the present invention is a compound which has improved hygroscopicity and stability under increased humidity conditions, and remarkably enhanced stability against light, as compared to the hydrochloride which is a known compound, and which is very useful as a medicament or a drug substance.
Particularly, as generally known, the medicament or a bulk for the medicament having improved hygroscopicity and stability under increased humidity conditions has reduced problems in the humid storage condition and in quality control and also, for the production of a solid formulation such as a tablet, a capsule, and the like, it is known that the problems in production based on the change in the weight of the effective components are reduced. That is, the specific acid addition salt of the compound A of the present invention has improved hygroscopicity and stability under increased humidity conditions, and thus, it is a compound that stable storage and easy quality maintenance are expected and can be easily handled in the production of formulations, and therefore, it contributes to provision of an excellent medicament with higher quality.

### Brief Description of Drawings

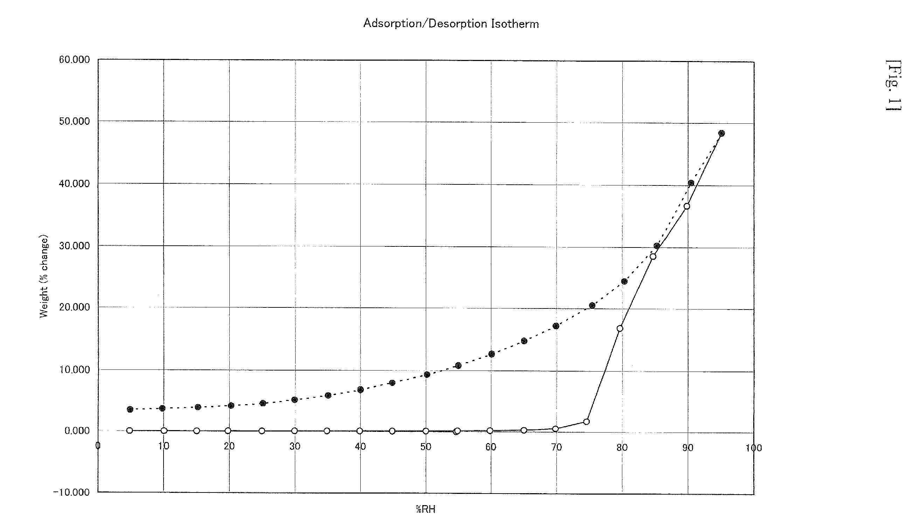

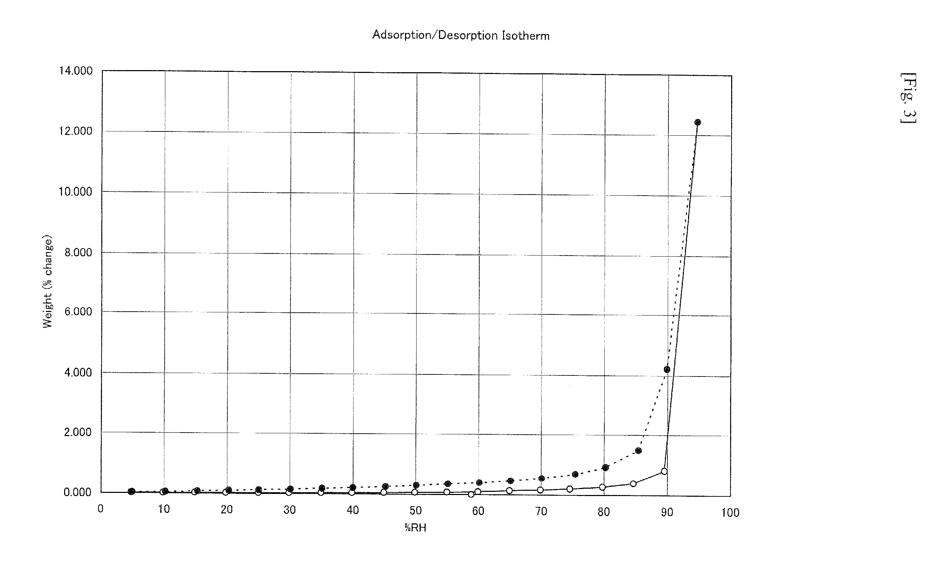

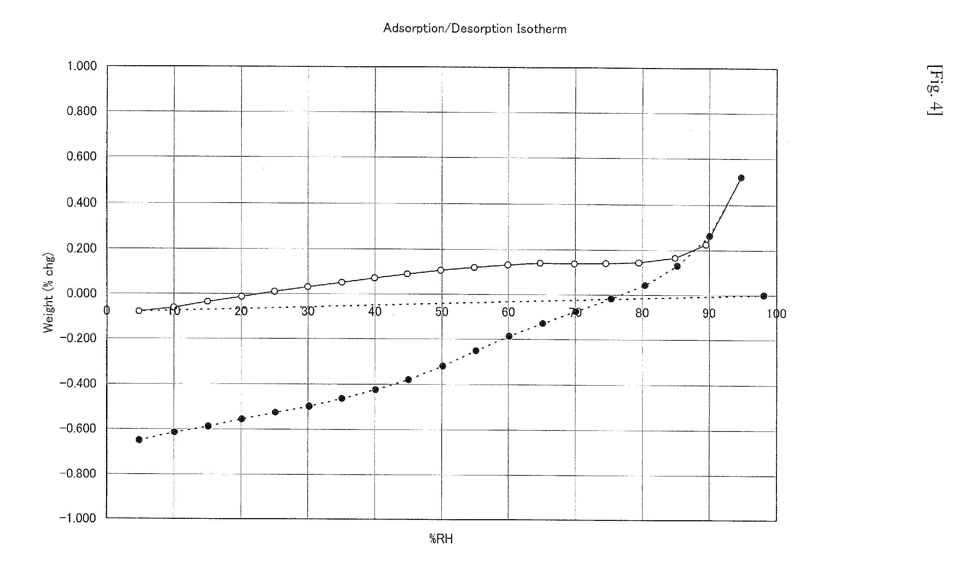

[Fig. 1] Fig. 1 is a graph showing an isothermal curve of water absorption and desorption of the hydrochloride of the compound A which is a known compound.
[Fig. 2] Fig. 2 is a graph showing an isothermal curve of water absorption and desorption of the compound of Example 1.
[Fig. 3] Fig. 3 is a graph showing an isothermal curve of water absorption and desorption of the compound of Example 2.
[Fig. 4] Fig. 4 is a graph showing an isothermal curve of water absorption and desorption of the compound of Example 4.
   Furthermore, in Figs. 1 to 4, the ordinate indicates the change in weight at each relative humidity with respect to the weight of the compound A at a time of measurement initiation, in terms of a ratio (%) to the weight of the compound A at a time of measurement initiation. The abscissa indicates the relative humidity (%). The curve of the white circle-solid line represents an absorption isothermal curve and the curve of the black circle-dotted line represents a desorption isothermal curve.

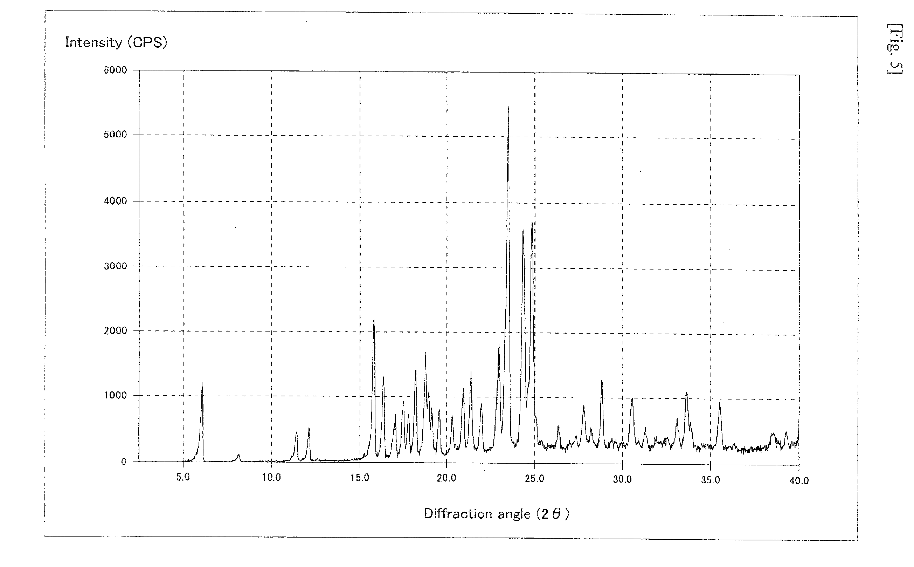

[Fig. 5] Fig. 5 is a graph showing a powder X-ray diffraction pattern of the compound of Example 1.

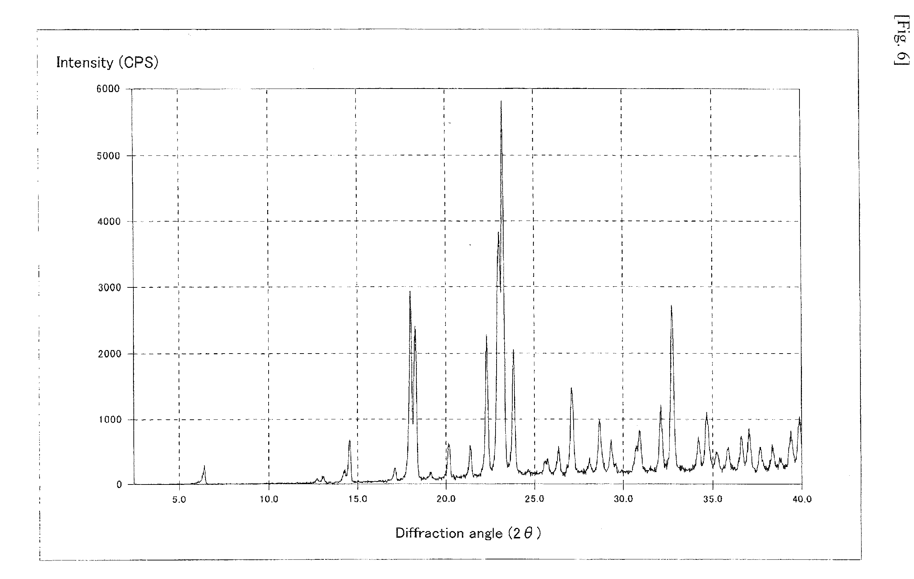

[Fig. 6] Fig. 6 is a graph showing a powder X-ray diffraction pattern of the compound of Example 2.

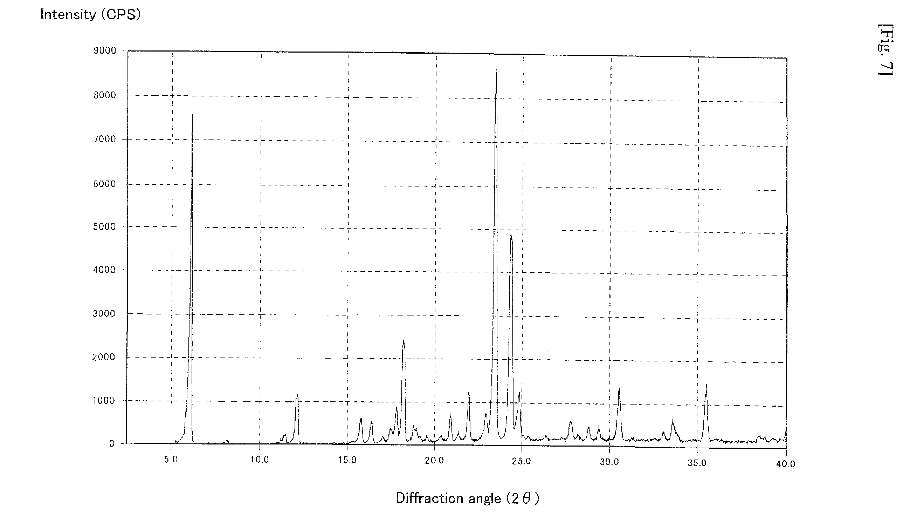

[Fig. 7] Fig. 7 is a graph showing a powder X-ray diffraction pattern of the compound of Example 3.

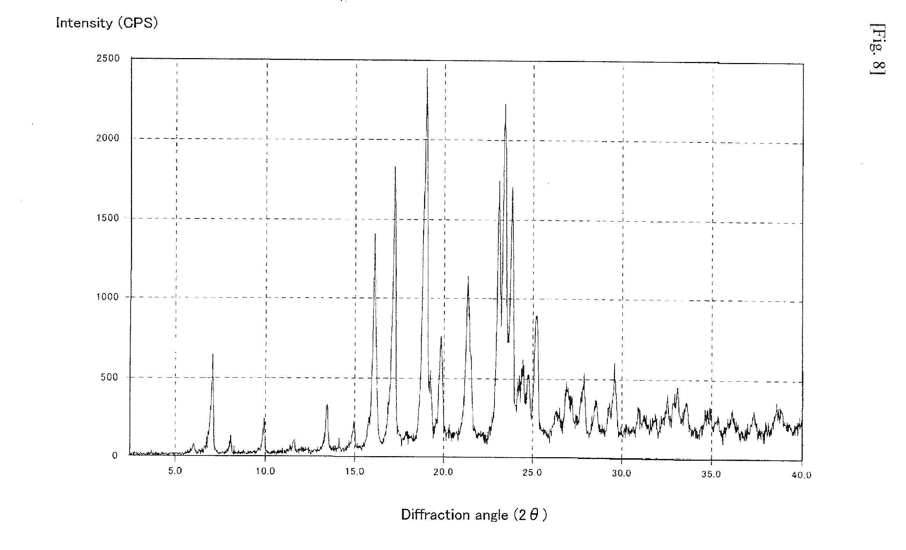

[Fig. 8] Fig. 8 is a graph showing a powder X-ray diffraction pattern of the compound of Example 4.

### Best Mode for Carrying Out the Invention

The specific acid addition salt of the compound A of the present invention is stable under humidity so that it can be used as a medicament or a drug substance, and particularly when it is used as a medicament or a drug substance, it has no problematic hygroscopicity and it is chemically stable or expected to be stable under storage. Accordingly, any acid addition salt of the present invention is suitable as a medicament or a drug substance, particularly as a drug substance of a solid formulation, and it is particularly preferably an acid addition salt of the compound A with benzenesulfonic acid.

Preferred embodiments of the present invention are shown below.
(1) A crystal of a compound A benzenesulfonate.
(2) The crystal as described in (1), in which the endothermic onset temperature in DSC is around 139 to 141 °C,
(3) The crystal as described in (1), wherein in the powder X-ray analysis using Cu as a radiation source, 2θ (°) exhibits peaks at around 6.1, around 15.8, around 23.5, and around 24.8.
(4) The crystal as described in (1), wherein in the powder X-ray analysis using Cu as a radiation source, 2θ (°) exhibits peaks at around 6.1, around 8.1, around 11.4, around 12.1, around 15.8, around 23.5, and around 24.8.
(5) The crystal as described in (1), wherein the endothermic onset temperature in DSC is 139 to 141°C, and in the powder X-ray analysis using Cu as a radiation source, 2θ (°) exhibits peaks at around 6.1, around 15.8, around 23.5, and around 24.8.
(6) The crystal as described in (1), wherein the endothermic onset temperature in DSC is 139 to 141°C, and in the powder X-ray analysis using Cu as a radiation source, 2θ (°) exhibits peaks at around 6.1, around 8.1, around 11.4, around 12.1, around 15.8, around 23.5, and around 24.8.
(7) The crystal as described in any one of (1) to (6), which is an α-form crystal.
(8) The crystal as described in (1), wherein the endothermic onset temperature in DSC is around 137 to 139°C.
(9) The crystal as described in (1), wherein in the powder X-ray analysis using Cu as a radiation source, 2θ (°) exhibits peaks at around 7.1, around 10.0, around 17.3, and around 23.3.
(10) The crystal as described in (1), wherein in the powder X-ray analysis using Cu as a radiation source, 2θ (°) exhibits peaks at around 7.1, around 8.1, around 10.0, around 13.5, around 17.3, around 19.1, and around 23.3.
(11) The crystal as described in (1), wherein the endothermic onset temperature in DSC is 137 to 139°C, and in the powder X-ray analysis using Cu as a radiation source, 2θ (°) exhibits peaks at around 7.1, around 10.0, around 17.3, and around 23.3.
(12) The crystal as described in (1), wherein the endothermic onset temperature in DSC is 137 to 139°C, and in the powder X-ray analysis using Cu as a radiation source, 2θ (°) exhibits peaks at around 7.1, around 8.1, around 10.0, around 13.5, around 17.3, around 19.1, and around 23.3.
(13) The crystal as described in any one of (1), and (8) to (12), which is a β-form crystal.
(14) A pharmaceutical composition comprising a compound A benzenesulfonate as an active ingredient, and further a pharmaceutically acceptable carrier.
(15) The pharmaceutical composition as described in (14), wherein the compound A benzenesulfonate is the crystal as described in any one of (1) to (13).

(16) A crystal of a compound A hydrobromide.
(17) The crystal as described in (16), in which the endothermic onset temperature in DSC is around 165 to 167°C.
(18) The crystal as described in (16), in which in the powder X-ray analysis using Cu as a radiation source, 2θ (°) exhibits peaks at around 18.0, around 18.3, around 22.3, and around 23.2.
(19) The crystal as described in (16), in which in the powder X-ray analysis using Cu as a radiation source, 2θ (°) exhibits peaks at around 6.4, around 14.6, around 18.0, around 18.3, around 20.2, around 22.3, and around 23.2.
(20) The crystal as described in (16), in which the endothermic onset temperature in DSC is around 165 to 167°C, and in the powder X-ray analysis using Cu as a radiation source, 2θ (°) exhibits peaks at around 18.0, around 18.3, around 22.3, and around 23.2.
(21) The crystal as described in (16), in which the endothermic onset temperature in DSC is around 165 to 167°C, and in the powder X-ray analysis using Cu as a radiation source, 2θ (°) exhibits peaks at around 6.4, around 14.6, around 18.0, around 18.3, around 20.2, around 22.3, and around 23.2.
(22) The crystal as described in any one of (16) to (21), which is an α-form crystal.
(23) A pharmaceutical composition including the compound A hydrobromide as an active ingredient, and further a pharmaceutically acceptable carrier.
(24) The pharmaceutical composition as described in (23), wherein the compound A benzenesulfonate is the crystal as described in any one of (16) to (22).

Furthermore, in the powder X-ray diffraction pattern, due to the nature of the data, the diffraction angles or entire patterns are important for verification of the identity of a crystal, the relative intensity varies more or less depending on the orientation of the crystal growth, the particle size, and the measurement conditions, and accordingly it should not be strictly interpreted. Furthermore, the present invention encompasses a pure α-form crystal and a pure β-form crystal of the compound A benzenesulfonate, and a pure α-form crystal of the compound A hydrobromide, and a mixture that is considered essentially equivalent to these pure crystals within the present invention.

The values obtained from various patterns may have some errors resulted from the orientation of the crystal growth, the particle size, and the measurement conditions in some cases. Accordingly, in the present specification, the term "around" as used in the values of the diffraction angles (2θ) in the powder X-ray diffraction pattern largely means that it is almost the values, and preferably, it means that the values may be not more or less than the values by 0.2 (°). More preferably, it means that the values may be not more or less than the values by 0.1 (°).

Furthermore, the term ``around" as used in the values of the endothermic onset temperature in DSC largely means the values of the temperature of the endothermic onset (extrapolation initiation), preferably, it means that the values may be not more or less than the values by 2°C, and more preferably, it means that the values may be not more or less than the values by 1°C.

The "the α-form crystal of the compound A benzenesulfonate" of the present invention can be obtained by any one of the following method A and method B.

### Method A

1) The compound A, (-)-(2R,3R)-di-O-benzoyl tartrate, is dissolved in a suitable solvent, and 2) neutralized with a suitable base, 3) benzenesulfonic acid is added to the solution, and 4) the compound A benzenesulfonate is crystallized with a suitable solvent.

### Method B

1) The Boc group oftert-butyl (R)-2-[(1H-inden-7-yloxy)methyl]morpholine-4-carboxylate is deprotected with benzenesulfonic acid, and 2) crystallization is carried out with a suitable solvent.

When the compound A exists as a free form in a solvent, isomerization of a double bond on the indene ring easily occurs, and thus, the Method A is required to be carried out, preferably 1) under ice-cooling, 2) with a weak base such as sodium hydrogen carbonate and the like, and 3) for as short a time as possible. As the "suitable solvent in which the compound A (-)-(2R,3R)-di-O-benzoyl tartrate is dissolved" used in the Method A is not particularly limited as long as it is an inert solvent not having an effect on the stability of the compound, and examples thereof include alcohols such as methanol, ethanol, 2-propanol, and the like, ethers such as diethyl ether, tetrahydrofuran, dioxane, dimethoxyethane, and the like, ethyl acetate, N,N-dimethylformamide, dimethylsulfoxide, water, and a mixture thereof. The base for neutralization is not particularly limited as long as it is a base not having an effect on the stability of the compound, but preferable examples thereof include sodium hydrogen carbonate.

The benzenesulfonic acid used in the deprotection of Boc group of the Method B is used at 1.0 to 1.1 equivalents based on the tert-butyl (R)-2-[(1H-inden-7-yloxy)methyl]morpholine-4-carboxylate, and stirred in a solvent inert to the reaction at between room temperature and 80°C, usually for 0.1 hours to 5 hours. Examples of the solvent used herein are not particularly limited, but include alcohols such as methanol, ethanol, 2-propanol, and the like, ethers such as diethyl ether, tetrahydrofuran, dioxane, dimethoxyethane, and the like, ketones such as acetone, methylethylketone, and the like, hydrocarbons such as toluene and the like, ethyl acetate, N,N-dimethylformamide, dimethylsulfoxide, and a mixture thereof.

Examples of the solvent for crystallization in the Methods A and B include alcohols such as methanol, ethanol, 2-propanol, and the like, ethers such as diethyl ether, tetrahydrofuran, dioxane, dimethoxyethane, and the like, ketones such as acetone, methylethylketone, and the like, hydrocarbons such as toluene and the like, ethyl acetate, and a mixed solvent thereof. That is, when crystallization of the compound A benzenesulfonate is carried out using the solvent, an α-form crystal of the compound A benzenesulfonate can be obtained.

In the Method B, after the deprotection of Boc group, crystallization may also be carried out without evaporation of the reaction solvent. Examples of the reaction solvent (that is, the crystallization solvent) in this case include alcohols such as methanol, ethanol, 2-propanol, and the like, ethers such as diethyl ether, tetrahydrofuran, dioxane, dimethoxyethane, and the like, ketones such as acetone, methylethylketone, and the like, hydrocarbons such as toluene and the like, ethyl acetate, and a mixture thereof.

The "β-form crystal of the compound A benzenesulfonate" of the present invention can be obtained by controlling the crystallization conditions of the Method B above. That is, by choosing the crystallization conditions in the Method B, the α-form crystal and the β-form crystal of the "compound A benzenesulfonate" can be respectively prepared.

First, in the case that the α-form crystal of the compound A benzenesulfonate is subjected to a Boc-elimination reaction, followed by evaporation of the solvent, and then crystallization is carried out, the conditions for the crystallization are as follows. That is, the compound A benzenesulfonate is added to a specific solvent at an amount giving a concentration that is 1/10 or less of the solubility of the "compound A benzenesulfonate α-form crystal" after warming, and then warmed. Then, it is cooled over one hour or longer to a temperature at which the compound A benzenesulfonic acid is oversaturated, thereby obtaining the α-form crystal of the compound A benzenesulfonate. Preferably, when ethyl acetate is used as the crystallization solvent, the compound A benzenesulfonate is added in an amount giving a concentration of 0.01 mol/l or less, and the compound A benzenesulfonate is dissolved at 70°C or higher, and cooled to 15°C to 25°C.

The conditions when the α-form crystal of the compound A benzenesulfonate is obtained by carrying out crystallization without evaporation of the solvent after the deprotection of Boc group are as follows. That is, when ethyl acetate is used as a solvent for a reaction/crystallization, tert-butyl (R)-2-[(1H-inden-7-yloxy)methyl]morpholine-4-carboxylate and benzenesulfonic acid can be used and reacted in the amounts such that the concentration of the resulting compound A benzenesulfonate after the deprotection of Boc group is 0.3 mol/l or less at a temperature of 70°C or higher, and then cooled to 15°C to 25°C over 1 hour or more, thereby obtaining an α-form crystal of the compound A benzenesulfonate. Alternatively, when a mixed solvent of acetone-toluene is used as a solvent for a reaction/crystallization, tert-butyl (R)-2-[(1H-inden-7-yloxy)methyl]morpholine-4-carboxylate and benzenesulfonic acid can be used and reacted in the amounts such that the concentration of the resulting compound A benzenesulfonate after the deprotection of Boc group is 0.4 mol/l or less at a temperature of 80°C or higher, and then cooled to 20°C to 30°C over 1 hour or more, thereby obtaining the α-form crystal.

When the β-form crystal of the compound A benzenesulfonate is obtained, tert-butyl (R)-2-[(1H-inden-7-yloxy)methyl]morpholine-4-carboxylate and benzenesulfonic acid can be preferably used and reacted in the amounts such that the concentration of the resulting compound A benzenesulfonate after the deprotection of Boc group is 0.4 mol/l or more at a temperature of 70°C or higher, and then cooled rapidly to -50°C or lower within 10 minutes or less, thereby obtaining the β-form crystal.

Depending on the crystallization conditions for Method B other than the above, mixed crystals of the α-form crystal and the β-form crystal of the "compound A benzenesulfonate" may be obtained in some cases. In this case, the β-form crystal in the mixture can be converted into the α-form crystal by stirring the suspension of the mixture in a solvent inert to the reaction at between room temperature and 70°C, usually for 0.1 hours to 1 day. Examples of the solvent used herein are not particularly limited, but include alcohols such as methanol, ethanol, 2-propanol, and the like, ketones such as acetone, methylethylketone, and the like, ethers such as diethyl ether, tetrahydrofuran, dioxane, dimethoxyethane, and the like, hydrocarbons such as toluene and the like, ethyl acetate and a mixture thereof.

In the present specification, specific examples of the "pharmaceutically acceptable acid" include specific organic acid salts (acetate, malonate, fumarate, tartrate, methane sulfonate, benzenesulfonate, formate, toluenesulfonate, trifluoroacetate, citrate, adipate, benzoate, gluconate, glucronate, pamoate, and the like), specific inorganic acid salts (hydrochloride, hydrobromide, sulfate, phosphate, nitrate, and the like), amino acid salts (argininate, aspartate, glutamate, and the like), etc.

The acid addition salts of the compound A of the present invention can be used as a drug substance by combining one or more of the acid addition salts of the compound A of the present invention with a pharmaceutically acceptable carrier, and excipient, or the like, to prepare the medicament. The preparation of the medicament can be carried out by a method usually employed in this field.
The medicament containing the acid addition salt of the compound A of the present invention may take any form of preparation for oral administration, such as tablets, pills, capsules, granules, powders, liquids and solutions, and the like; or preparations for parenteral administration, such as intraarticular, intravenous, or intramuscular injections, and the like, suppositories, percutaneous liquid preparations, ointments, transdermal patches, transmucosal liquid preparations, transmucosal patches, inhalations, and the like. Particularly, the preparations for oral administration containing the crystal of the addition salt of the compound A, such as tablets, pills, capsules, granules, and powders, are advantageous as stable solid formulations.

In the solid compositions for oral administration, one or more of the active ingredients may be mixed with at least one inert diluent, for example, lactose, mannitol, glucose, hydroxypropylcellulose, microcrystalline cellulose, starch, polyvinylpyrrolidone, magnesium metasilicate aluminate, and the like. The compositions may contain additives other than inert diluents in a conventional manner, for example, lubricants such as magnesium stearate and the like, disintegrating agents such as fibrous calcium glycolate and the like, stabilizers, solubilizing agents, etc. The tablets or pills may be coated, if required, with sugar-coating or a gastric or enteric coating film, such as sucrose, gelatin, hydroxypropyl cellulose, hydroxypropylmethyl cellulose phthalate, and the like.

Examples of the liquid compositions for oral administration include pharmaceutically acceptable emulsions, solutions, suspensions, syrups, elixirs, and the like, which contain conventionally used inert diluents, for example, purified water or ethanol. In addition to inert diluents, the compositions may further contain auxiliary agents such as a wetting agent and a suspending agent, a sweetener, a flavor, an aromatic, or a preservative.
Examples of the injections for parenteral administration include sterile aqueous or non-aqueous solutions, suspensions and emulsions. The aqueous solutions and suspensions include, for example, distilled water for injection and physiological saline. Examples of the non-aqueous solutions and suspensions include propylene glycol, polyethylene glycol, vegetable oils such as olive oil and the like, alcohols such as EtOH and the like, polysorbate 80, etc. Such composition may further contain auxiliary agents such as a preservative, wetting agent, emulsifying agent, dispersant, stabilizer, solubilizing agent, or the like. These may be sterilized, for example, by filtration through a bacterium-impermeable filter, blending with a bactericide, or irradiation. Alternatively, these may be made into a sterile solid composition, which is dissolved in sterile water or sterile solvent for injection just before use.

Since the pharmaceutical compositions of the present invention contain the acid addition salt of the compound A of the present invention, which is an SNRI, as an active ingredient, it can be provided for treatment or prevention of various diseases in which SNRI can be used. That is, the pharmaceutical composition of the present invention is specifically useful as an agent for treating psychiatric symptoms such as depression, post-stroke depression, emotional disorders, reduced motivation, and the like; or as an agent for treating or preventing neuropathic pain, such as allodynia, hyperalgesia, hyperesthesia, spontaneous pain, cancer pain, trigeminal neuralgia, phantom limb pain, postherpetic neuralgia, fibromyalgia, low back/leg pain, thalamic pain, entrapment (compressive) peripheral neuropathy, pain accompanying central neuropathy, atypical facial pain, pain accompanying spinal cord injury, pain accompanying multiple sclerosis, pain accompanying chemotherapy-induced neuropathy, cancer pain on which an analgesic effect of a narcotic analgesic such as morphine and the like is insufficient, pain accompanying diabetic neuropathy, and the like.

The pharmaceutical agent used in the present invention may be administered to a patient with psychiatric symptoms such as depression, post-stroke depression, emotional disorders, reduced motivation, and the like, or neuropathic pain, and in oral administration, the daily dose is usually suitably from about 0.001 to 100 mg/kg per body weight, and this is administered in one portion or dividing it into 2 to 4 portions. In the case of intravenous administration, the daily dose is suitably from about 0.0001 to 10 mg/kg per body weight, and this is administered once a day or two or more times a day. In addition, a transmucosal agent is administered at a dose from about 0.001 to 100 mg/kg per body weight, and this is administered once a day or two or more times a day. The dose is appropriately decided in response to an individual case by taking the symptoms, the age, the gender, and the like into consideration.

### Examples

The following Examples are for the purpose of illustrating the present invention in further detail and are not intended to limit the present invention. The invention is fully illustrated by way of the Examples, however, it is understood that it will be apparent to those skilled in the art that various modifications and variations of the Examples can be made therein. Accordingly, such modifications and variations are included in the present invention as long as they do not depart from the scope of the present invention.

Hereinbelow, the present invention is described in detail with reference to Examples, but the present invention is not intended to be limited by Examples, and the scope of the present invention is not limited thereto.
Further, the thermal analysis and powder X-ray diffractometry were performed according to the following methods.

### (1) Thermal Analysis (DSC)

Approximately 3 mg of a sample was placed in an exclusively-used aluminum-made sample pan, and the change in calories generated between the sample and a reference (an empty aluminum-made sample pan), with a measurement range from room temperature to 300°C under a nitrogen atmosphere (50 mL/min) and a temperature elevation rate of 10°C/min were continuously measured and recorded. Furthermore, the handling of the devices including data processing was conducted in accordance to the methods and procedures as indicated in each device. (Device: Hi-Res DSC 2910, manufactured by TA Instrument).

### (2) Powder X-Ray Diffractometry

Approximately 10 mg of a sample was placed in an exclusively-used sample holder (width 5 mm, length 18 mm, and depth 0.2 mm), and the powder X-ray diffraction pattern of the sample was continuously measured and recorded under the following conditions. Furthermore, the handling of the devices including data processing was conducted in accordance to the methods and procedures as indicated in each device. (Device: MXP18TAHF22 manufactured by MAC Science (present name: Bruker).

### (Conditions)

Radiation source: Cu; wavelength: 1.54056 Å, measurement range: 2.50 to 40.00°, sampling interval: 0.02°, scanning rate: 4.00°/min, tube voltage: 40 kV, tube current: 200 mA, divergence slit: variable (radiation width 5.00 mm), scattering slit: variable (radiation width 5.00 mm), receiving slit: 0.15 mm.
Furthermore, the diffraction angle and diffraction intensity may vary more or less depending on the orientation of the crystal growth, the particle size, the measurement conditions, and the like. Accordingly, the numeral values thereof should not be strictly solved.

### Reference Example 1 Preparation of the racemic hydrochloride (indeloxazine hydrochloride) of the compound A, which is a starting material compound of Reference Examples 2 and 3

Preparation was conducted in accordance with the method as described in the specification of U.S. Patent No. 4109088.

### Reference Example 2 Preparation of Compound A (-)-(2R,3R)-di-O-benzoyl tartrate, which is a raw material compound of Examples 1 and 2

Preparation was carried out using the racemic hydrochloride of the compound A obtained in Reference Example 1 as a starting material in accordance with the method as described in Chemical and Pharmaceutical Bulletin (1985, Vol. 33, No. 9, p. 3766-3774).

### Reference Example 3 Preparation of the compound A hydrochloride, which is a comparative compound

Preparation was conducted using the racemic hydrochloride of the compound A obtained in Reference Example 1 as a starting material in accordance with the method as described in Chemical and Pharmaceutical Bulletin (1985, Vol. 33, No. 9, p. 3766-3774).

### Preparation Example 1

59.26 g of 4-hydroxyindan-1-one and 148.58 g of tert-butyl (R)-2-[(tosyloxy)methyl]morpholine-4-carboxylate were dissolved in 415 mL of N-methylpyrrolidone at room temperature. 138.21 g of potassium carbonate fine powders was added to the solution , followed by thoroughly stirring at 100°C to perform a reaction for 5 hours. After returning to room temperature, 831 mL of water was added dropwise to the reaction mixture over 30 minutes or more to allow crystals to precipitate. After stirring overnight, the crystals were collected by filtration, washed with 295 mL of water, and then dried under reduced pressure at 50°C overnight to obtain 134.72 g of tert-butyl (R)-2-{[(1-oxoindan-4-yl)oxy]methyl}morpholine-4-carboxylate as a pale brown crystal.
¹H-NMR (CDCl₃): 1.48 (9H, s), 2.68 (2H, m), 2.80-3.15 (4H, m), 3.61 (1H, m), 3.80-4.20 (6H, m), 7.03 (1H, d, J=7.6Hz), 7.30-7.45 (2H, m)
MS (ES+): 370.1 [M+Na]⁺

### Preparation Example 2

30.0 g oftert-butyl (R)-2-{[(1-oxoindan-4-yl)oxy]methyl}morpholine-4-carboxylate ester was dissolved in 300 mL of methanol, and 3.27 g of sodium borohydride was added to the solution, followed by stirring at room temperature for 1 hour and 45 minutes. To the reaction solution was added 150 mL of a saturated aqueous ammonium chloride solution, followed by stirring for 10 minutes, and 150 mL of water was added to the solution, followed by extraction with 300 mL of ethyl acetate twice. The resulting organic layer was combined and concentrated under reduced pressure at 40°C to obtain a brown oily residue. The oily residue was dissolved in 300 mL of ethyl acetate, washed with 300 mL of water, 330 mL of brine (300 mL of water+30 mL of saturated brine), and concentrated under reduced pressure at 40°C to obtain 30.42 g of tert-butyl (R)-2-{[(1-hydroxyindan-4-yl)oxy]methyl}morpholine-4-carboxylate ester as a brown oily substance.
¹H-NMR (CDCl₃): 1.48 (9H, s), 1.87-2.00 (1H, m), 2.43-2.57(1H, m), 2.70-3.15 (4H, m), 3.55-3.66 (1H, m), 3.73-4.20 (6H, m), 5.23 (1H, m), 6.75 (1H, d, J=7.6Hz), 7.05 (1H, d, J=7.6Hz), 7.21 (1H, t, J=7.6Hz)
MS (EI): 372.2 [M+Na]⁺

### Preparation Example 3

9.63 g of tert-butyl (R)-2-{[(1-hydroxyindan-4-yl)oxy]methyl}morpholine-4-carboxylate was dissolved in 576 mL of 1,2-dichloroethane, and 5.23 g of p-toluenesulfonic acid monohydrate was added to the solution, followed by heating under reflux for 30 minutes. After returning to room temperature, 6.0 g of di-tert-butyl dicarbonate and a 27.5 mL of 1 M aqueous sodium hydrogen carbonate solution were added to the reaction mixture, followed by thoroughly stirring at room temperature for 30 minutes. 100 mL of water was added to the solution, followed by stirring, and then an organic layer was collected by separation. Further, to the aqueous layer was added 100 mL of 1,2-dichloroethane, followed by stirring, and then the organic layer was collected by separation. The obtained organic layer was combined and concentrated under reduced pressure at 40°C to obtain a brown oily residue. The obtained oily residue was dissolved in 200 mL of ethyl acetate, followed by washing with 100 mL of water and 50 mL of water. The organic layer was concentrated under reduced pressure at 40°C to obtain 9.57 g of a brown oily substance.
The brown oily substance obtained by the same method was combined to give 46.58 g of a brown oily substance, which was purified by silica gel column chromatography (heptane:ethyl acetate=3:1) to obtain 30.64 g of tert-butyl (R)-2-[(1H-inden-7-yloxy)methyl]morpholine-4-carboxylate as a pale yellow oily substance.
¹H-NMR (CDCl₃): 1.48 (9H, s), 2.80-3.10 (2H, m), 3.39 (2H, s), 3.60 (1H, m), 3.75-4.25 (6H, m), 6.56 (1H, m), 6.73 (1H, d, J=7.6Hz), 6.84 (1H, m), 7.07 (1H, d, J=7.6Hz), 7.24 (1H, t, J=7.6Hz)
MS (ES+): 355.0 [M+Na]⁺

### Example 1 Preparation of the compound A benzenesulfonate (α-form crystal)

1000 mg of compound A (-)-(2R,3R)-di-O-benzoyl tartrate was added to 20 ml of ethyl acetate-20 ml of a saturated aqueous sodium hydrogen carbonate solution under ice-cooling, followed by stirring rapidly under the same conditions, and then the organic layer was collected by separation. The obtained organic layer was washed rapidly twice with 20 ml of an ice-cooled saturated aqueous sodium hydrogen carbonate solution, then washed rapidly with 10 ml of ice-cooled saturated brine, dried rapidly over anhydrous magnesium sulfate and added to a solution of 269 mg of benzenesulfonic acid in 10 ml of ethanol, which had been stirred rapidly under ice-cooling. The resulting solution was concentrated under reduced pressure at room temperature or lower, and then crystallized with ethanol-diethyl ether. The crystals were collected by filtration and washed with diethyl ether. The resulting crystal was recrystallized from 3 ml of acetone-0.1 ml of water-3 ml of diethyl ether to obtain 422 mg of the compound A benzenesulfonate as a white crystal (α-form crystal).
¹H-NMR (DMSO-d₆): 2.96-3.10 (2H, m), 3.20-3.44 (4H, m), 3.69-3.79 (1H, m), 3.99-4.09 (2H, m), 4.11-4.19 (2H, m), 6.59-6.64 (1H, m), 6.84 (1H, d, J=8.0Hz), 6.88-6.93 (1H, m), 7.08 (1H, d, J=7.6Hz), 7.20-7.36 (4H, m), 7.56-7.63 (2H, m), 8.85 (2H, bs)
MS (EI): 231.0
Elemental Analysis: Calcd. Values (for C14H17NO2.C6H6O3S) C 61.68%, H 5.95%, N 3.60%, S 8.23%; Found Values C 61.60%, H 5.92%, N 3.47%, S 8.23%
Endothermic onset temperature in DSC: 140°C
The powder X-ray diffraction pattern of the compound of Example 1 is shown in Fig. 5.

### Example 2 Preparation of the compound A hydrobromide (α-form crystal)

438 mg of 47% hydrobromic acid was dissolved in a mixed solvent of 75 ml of water-75 ml of methanol at room temperature. While stirring under ice-cooling, 150 ml of diethyl ether and then 1500 mg of the compound A (-)-(2R,3R)-di-O-benzoyl tartrate were added to the solution, followed by stirring for 16 hours under the same conditions. The reaction mixture was allowed to stand, and the aqueous layer was collected by separation and washed four times with 75 ml of diethyl ether. The resulting aqueous layer was concentrated under reduced pressure at room temperature or lower, and azeotroped with toluene at room temperature or lower. The resulting oily substance was crystallized with isopropanol-diethyl ether, and then the crystals were collected by filtration. The resulting crystals were recrystallized from 8 ml of ethanol-10 ml of diethyl ether to obtain 505 mg of the compound A hydrobromide as a white crystal (α-form crystal).
¹H-NMR (DMSO-d₆): 2.95-3.11 (2H, m), 3.16-3.46 (4H, m), 3.70-3.82 (1H, m), 3.98-4.21 (4H, m), 6.58-6.65 (1H, m), 6.80-6.94 (2H, m), 7.08 (1H, d, J=7.6Hz), 7.24 (1H, t, J=7.6Hz), 8.94 (2H, bs)
MS (ESI⁺): 232.1
Elemental Analysis: Calcd. Values (for C14H17NO2.HBr) C 53.86%, H 5.81%, N 4.49%, Br 25.59%; Found Values C 53.45%, H 5.65%, N 4.43%, Br 25.44%
Endothermic onset temperature in DSC: 166°C
The powder X-ray diffraction pattern of the compound of Example 2 is shown in Fig. 6.

### Example 3 Preparation of the compound A benzenesulfonate (α-form crystal)

7.0 g of tert-butyl (R)-2-[(1H-inden-7-yloxy)methyl]morpholine-4-carboxylate and 3.34 g of benzenesulfonic acid were dissolved in 165 mL of ethyl acetate, followed by stirring at 70°C for 5 hours. The reaction solution in which the crystals were precipitated was cooled to room temperature, followed by stirring at room temperature overnight. The crystals were collected by filtration, washed twice with 7 mL of ethyl acetate, and then dried under reduced pressure at 50°C to obtain 6.94 g of (R)-2-[(1H-inden-7-yloxy)methyl]morpholine benzenesulfonate (α-form crystal) as a white crystal.
¹H-NMR (DMSO-d₆): 2.96-3.10 (2H, m), 3.20-3.44 (4H, m), 3.69-3.79 (1H, m), 3.99-4.09 (2H, m), 4.11-4.19 (2H, m), 6.59-6.64 (1H, m), 6.84 (1H, d, J=8.0Hz), 6.88-6.93 (1H, m), 7.08 (1H, d, J=7.6Hz), 7.20-7.36 (4H, m), 7.56-7.63 (2H, m), 8.91 (2H, bs)
MS (ES+): 232.1
Elemental Analysis: Calcd. Values (for C14H17NO2.C6H603S) C 61.68%, H 5.95%, N 3.60%, S 8.23%; Found Values C 61.58%, H 5.88%, N 3.58%, S 8.05%
Endothermic onset temperature in DSC: 141°C
The powder X-ray diffraction pattern of the compound of Example 3 is shown in Fig. 7.

For the absolute configuration of the 2-[(1H-inden-7-yloxy)methyl]morpholine moiety in each of the compound A benzenesulfonate obtained in Example 1, the compound A hydrobromide obtained in Example 2, and the (R)-2-[(1H-inden-7-yloxy)methyl]morpholine benzenesulfonate obtained in Example 3, the identity was confirmed using a chiral column under the following conditions.

Agilent LC-1100 Series
Mobile phase: hexane/ethanol/diethylamine (400/600/1)
Column: CHIRALCEL OD-H (registered trademark) 0.46x25 cm
Column temperature: 40°C
Flow rate: 0.5 mL /min
Detection wavelength: 254 nm

It was confirmed that the retention time of the compound A ((+)-2-[(inden-7-yloxy)methyl]morpholine) hydrochloride obtained by the method described in Non-Patent Citation 1 under the above-described condition was 10 minutes and the retention time of the (-)-2-[(inden-7-yloxy)methyl]morpholine hydrochloride obtained by the above-described method was 23 minutes.

It was confirmed that the retention times of the compound A benzenesulfonate obtained in Examples 1 and 3, the compound A hydrobromide obtained in Example 2, and compound A hydrochloride obtained in Reference Example 3 were all 10 minutes, from which it can be seen that the (+)-2-[(inden-7-yloxy)methyl]morpholine has the same absolute configuration as that of the (R)-2-[(1H-inden-7-yloxy)methyl]morpholine.

### Example 4 Preparation of the compound A benzenesulfonate (β-form crystal)

10.0 g oftert-butyl (R)-2-[(1H-inden-7-yloxy)methyl]morpholine-4-carboxylate was dissolved in 37 ml of ethyl acetate, followed by heating and stirring at 80°C. A solution of 4.77 g of benzenesulfonic acid in 5 mL of ethyl acetate was added to the solution over 1 minute, followed by stirring for 3 minutes and then dropwise added to 30 mL of ethyl acetate, which had been cooled to -75°C. After stirring at -70°C or lower for 4 hours, the precipitated crystals were collected by filtration, washed with 20 mL of ethyl acetate, and then dried under reduced pressure at 50°C to obtain 6.52 g of a compound A benzenesulfonate (β-form crystal) as a white crystal.
¹H-NMR (DMSO-d₆): 2.96-3.10 (2H, m), 3.20-3.44 (4H, m), 3.69-3.79 (1H, m), 3.99-4.09 (2H, m), 4.11-4.19 (2H, m), 6.59-6.64 (1H, m), 6.84 (1H, d, J=8.0Hz), 6.88-6.93 (1H, m), 7.08 (1H, d, J=7.6Hz), 7.20-7.36 (4H, m), 7.56-7.63 (2H, m), 8.91 (2H, bs)
MS (ES+): 232.1
Elemental Analysis: Calcd. Values (for C14H17NO2.C6H6O3S) C 61.68%, H 5.95%, N 3.60%, S 8.23%; Found Values C 61.19%, H 5.87%, N 3.54%, S 8.13%
Endothermic onset temperature in DSC: 138°C
The powder X-ray diffraction pattern of the compound of Example 4 is shown in Fig. 8.

### Example 5 Preparation of the compound A benzenesulfonate (α-form crystal)

8 g of the compound A benzenesulfonate (α-form crystal) and 2 g of the compound A benzenesulfonate (β-form crystal) were suspended in 40 mL of acetone, and followed by stirring at 50°C for 5 hours. 30 mL of ethyl acetate was added dropwise to the suspension at 50°C over 30 minutes, and followed by stirring at 50°C for 2 hours and then stirring at 25°C for 16 hours. The crystals were collected by filtration, washed with 20 mL of ethyl acetate, and then dried under reduced pressure at 50°C to obtain 7.18 g of the compound A benzenesulfonate (α-form crystal) as a white crystal.

The effect of the acid addition salt of the compound A of the present invention was confirmed by the following Test Examples.

### Test Example 1 Evaluation of Hygroscopicity

Approximately 10 mg of a sample was placed in an exclusively-used quartz-made holder and measured under the conditions of a temperature: 25°C, a measurement range: relative humidity 5 to 95%, a measurement interval: relative humidity 5%. Furthermore, the handling of the devices including data processing was conducted in accordance to the methods and procedures as indicated in each device. (Device: dynamic steam adsorption/desorption measuring device SGA-100, manufactured by VTI).
The charts obtained in these tests are shown in Figs. 1 to 4. Further, the weight change within the range of measurement conditions is shown in Table 1.

### (Conditions)

Measuring temperature: 25°C; drying before measurement: not done; humidity at the beginning: 5% RH; maximum humidity: 95% RH; humidity at the end: 5% RH; step: 5% RH; equilibrium standard: 0.03 wt % in 5 min; maximum equilibrated time: 180 min; sampling interval: 20 sec; data recording interval: 1 min

**[Table 1]**

| Salt of the compound A (crystal form) | Weight change(%) |
|---|---|
| Benzenesulfonate (α) | <0.1 |
| Benzenesulfonate (β) | 1.2 |
| Hydrobromide (α) | 12.5 |
| Hydrochloride | 48.5 (accompanied by deliquescence) |

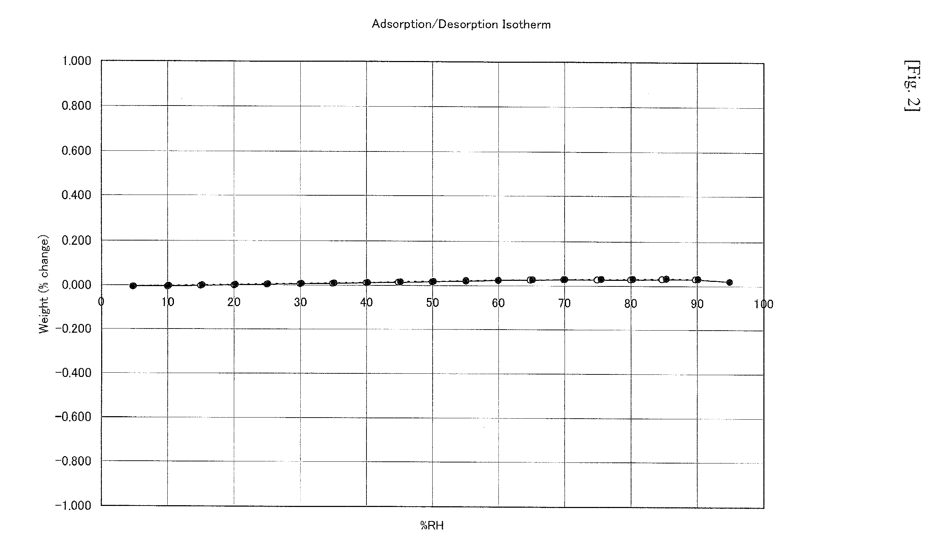

As shown in Fig. 1 and Table 1, the hydrochloride of the compound A, which is a known compound, becomes remarkably hygroscopic from a relative humidity of around 75%, and has a 50% increased weight change at a relative humidity of 5 to 95%, which is within a range of the measurement condition, and the change was accompanied by deliquescence. On the other hand, as shown in Figs. 2 to 4 and Table 1, the benzenesulfonate and hydrobromide of the compound A of the present invention have improved hygroscopicity, as compared to the hydrochloride of the compound A that is a known compound. Particularly, substantially no weight change was found for the benzenesulfonate in a range of the measurement condition; therefore, it was confirmed that it has excellent properties as a medicament or a drug substance.

### Test Example 2 Evaluation of Stability

Evaluation of the decomposed product during storage: Approximately 5 mg of a sample was metered into a 10 mL glass mass flask, and a test was carried out in the following storage conditions.
Condition 1: 40°C-relative humidity 75%-opening-3 months
Condition 2: 70°C-light shielding-sealing-2 weeks
Condition 3: 25°C-D65 (1000 lux)-sealing-2 weeks
A dissolution solvent was filled to a marked line in a mess flask including the sample after storage, the dissolved solution was taken as a sample solution, and the compound A in the sample solution was quantified. Furthermore, the detection of the compound A was carried out with UV at 254 nm and the handling of the devices including data processing was conducted in accordance to the methods and procedures as indicated in each device. (Device: LC-1100 Series manufactured by Agilent).
The results of these tests are shown in Table 2. Further, the quantitative value indicates the residual ratio of the compound A after the test to the compound A before the test.

**[Table 2]**

| Test condition | Quantitative Values (%) | | | |
|---|---|---|---|---|
| | Benzenesulfonate of α-form | Benzenesulfonate of β-form | Hydrobromide of α-form | Hydrochloride |
| Condition 1 | 101 | 95 | 98 | 55 |
| Condition 2 | 100 | 84 | 97 | 100 |
| Condition 3 | 94 | 74 | 69 | 52 |

As shown in Table 2, it is apparent that the hydrochloride of the compound A that is a known compound is not markedly stable against light and not stable under increased humidity conditions (Conditions 1 and 3). On the other hand, it was confirmed that benzenesulfonate of the compound A of the present invention has excellent stability against light and excellent stability under increased humidity conditions, as compared to the hydrochloride of the compound A that is a known compound, and thus has superior properties as a medicament or a drug substance.

### Test Example 3 Therapeutic effect of Compound A for Neuropathic Pain

The evaluation of an analgesic effect on neuropathic pain was performed according to the method of Kim et al. using L5/L6 spinal nerve ligation models. That is, the left L5 and L6 sciatic nerves of male SD rats were tightly ligated with a silk thread under pentobarbital anesthesia, and thereafter, the evaluation was performed. As the assessment of pain behavior, a von Frey Test on the plantar surface of the animals was employed (Pain, 1992, Vol. 50, p. 355-363). That is, the plantar surface of the operated limb of the nerve ligation rat was stimulated by using von Frey filaments until a withdrawal response was observed, and the lowest amount of force (g) required to elicit withdrawal response was defined as a pain threshold. The evaluation of compounds was performed in a period between 7 and 14 days after the operation in which a decrease in the threshold was stably observed. On the previous day of the evaluation of compounds, a von Frey Test was performed and the rats were grouped so as to minimize the variation in the mean value of pain threshold. Each of a solvent (vehicle) and a diluted solution obtained by diluting compound A hydrochloride as a compound A with a solvent so as to give a dose of 30 mg/kg was orally administered to rats in each group. Distilled water was used as a solvent. A pain threshold was measured at 1 hour after administration. An analgesic effect of the compound was examined based on recovery of the pain threshold in each compound administration group compared with that in the vehicle administration group, as S. K. Joshi et al. described (Neuroscience, 2006, Vol. 143, p. 587-596). A significant difference test was performed between the solvent administration group and the drug administration group using a Student's t-test.

### (Results)

The results of the von Frey Test are shown in Table 3. The numerical values in the table indicate a mean value±standard error (SEM) of recovery ratios of thresholds of the compounds when the pain threshold of the normal side paw is regarded as 100% and the pain threshold of the operated side paw is regarded as 0%. Compound A hydrochloride when orally administered at 30 mg/kg showed significant difference as compared to the solvent group. Furthermore, an effect that the compound improved the pain threshold in the operated side of paw to the same level as that in the normal paw was shown. That is, it was found that compound A hydrochloride has an analgesic action to restore the reduction in pain threshold relevant to neuropathic symptoms (abnormal paresthesia such as allodynia and the like) to a normal level.

**[Table 3]**

| | % Effect (30 mg/kg, p.o.) |
|---|---|
| Compound A hydrochloride | 94.5±17.0*** |

The symbol "***" in the table indicates that as a result of the Student's t-test, there is a significant difference, as compared to the vehicle group at a significance level less than 0.5%.

### Industrial Applicability

A specific acid addition salt of the compound A of the present invention is a compound which has improved hygroscopicity and stability under increased humidity conditions, and remarkable improved stability against light, as compared to the compound A hydrochloride which is a known compound, and thus extremely useful as a medicament or a drug substance.
That is, the specific acid addition salt of the compound A of the present invention has improved hygroscopicity and stability under increased humidity conditions, and therefore, it is a compound in which stable storage and easy quality control can be expected and can be easily handled during preparation, and therefore, it contributes to provision of a more excellent medicament with higher quality.

## Claims

1. A salt of (+)-2-[(inden-7-yloxy)methyl]morpholine with a pharmaceutically acceptable acid selected from a group consisting of benzenesulfonic acid and hydrobromic acid.

2. The salt according to claim 1, wherein the salt is (+)-2-[(inden-7-yloxy)methyl]morpholine benzenesulfonate.

3. A crystal of the salt according to claim 2.

4. The crystal according to claim 3, wherein the endothermic onset temperature in DSC is around 139 to 141°C.

5. The crystal according to claim 3, wherein in the powder X-ray analysis using Cu as a radiation source, 2θ (°) exhibits peaks at around 6.1, around 15.8, around 23.5, and around 24.8.

6. The crystal according to claim 3, 4, or 5, which is an α-form crystal.

7. The crystal according to claim 3, wherein the endothermic onset temperature in DSC is around 137 to 139°C.

8. The crystal according to claim 3, wherein in the powder X-ray analysis using Cu as a radiation source, 2θ (°) exhibits peaks at around 7.1, around 10.0, around 17.3, and around 23.3.

9. The crystal according to claim 3, 7, or 8, which is an β-form crystal.

10. The salt according to claim 1, wherein the salt is (+)-2-[(inden-7-yloxy)methyl]morpholine hydrobromide.

11. A crystal of the salt according to claim 10.

12. The crystal according to claim 11, wherein the endothermic onset temperature in DSC is around 165 to 167°C.

13. The crystal according to claim 11, wherein in the powder X-ray analysis using Cu as a radiation source, 2θ (°) exhibits peaks at around 18.0, around 18.3, around 22.3, and around 23.2.

14. The crystal according to claim 11, 12, or 13, which is an α-form crystal.
